# EUROPEAN PATENT APPLICATION

(11) **EP 3 447 044 A1**
(43) Date of publication of application: **27.02.2019**
(21) Application number: 17786103.6
(22) Date of filing: 05.04.2017
(51) Int. Cl.: C07C 213/08, C07C 219/22, C07C 213/10, C07B 55/00

(54) **METHOD FOR PREPARING D-4,4'-BIPHENYLALANINE ALKYL ESTER OR L-4,4'-BIPHENYLALANINE ALKYL ESTER FROM DL-4,4'-BIPHENYLALANINE ALKYL ESTER**

(30) Priority: 19.04.2016 KR 20160047462
(71) Applicant: Aminologics Co., Ltd., Seoul 06244 (KR)
(72) Inventor: BYUN, Il-Suk, Seongnam-si Gyeonggi-do 13587 (KR); YOO, Chul-Hwan, Daejeon 35330 (KR); KIM, Won-Sup, Sejong-si 30098 (KR)
(74) Representative: Cabinet Laurent & Charras
(86) International application number: PCT/KR2017/003744
(87) International publication number: WO 2017/183835

(57) **Abstract**

Disclosed is a method of preparing D-4,4'-biphenylalanine alkyl ester or L-4,4'-biphenylalanine alkyl ester by subjecting DL-4,4'-biphenylalanine alkyl ester to optical resolution using chiral diaroyl tartaric acid as an optical resolving agent.

## Description

### Technical Field

The present invention relates to a method of preparing D-4,4'-biphenylalanine alkyl ester or L-4,4'-biphenylalanine alkyl ester from DL-4,4'-biphenylalanine alkyl ester, and more particularly to a method of preparing D-4,4'-biphenylalanine alkyl ester or L-4,4'-biphenylalanine alkyl ester using chiral diaroyl tartaric acid as an optical resolving agent.

### Background Art

4,4'-biphenylalanine alkyl ester, represented by Chemical Formula 1 below, is a chiral compound having chiral carbon (chiral center). 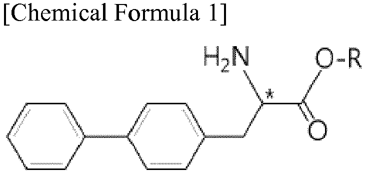
In Chemical Formula 1, * is chiral carbon (chiral center), and
R is a C1 to C4 alkyl group.

D-4,4'-biphenylalanine is a key material for use in the preparation of a neutral endopeptidase inhibitor, such as Sacubitril, serving as a therapeutic agent for heart failure, etc.

Conventionally, a method of preparing 4,4'-biphenylalanine having optical activity using a chiral hydrogenation catalyst (International Patent No. WO 2013/026773 (2013)) is problematic in that an expensive chiral hydrogenation catalyst has to be used and in that high-pressure hydrogen gas has to be used.

Furthermore, a method of preparing 4,4'-biphenylalanine having optical activity through a biological reaction using microorganisms is known. For example, there is known a method of preparing 4,4'-biphenylalanine from DL-N-Boc-4,4'-biphenylalanine methyl ester through hydrolysis using *Bacillus* sp. (European Patent No. 1,980,622 (2007)), which is disadvantageous because microorganisms that are difficult to handle from the aspect of biological processing must be used.

Also, there are known preparation methods that use Suzuki coupling by reacting a 4-substituted phenylalanine derivative having optical activity with phenylboronic acid in the presence of a palladium catalyst.

One of them is a preparation method in which a tyrosine derivative is introduced with a trifluorosulfonyl group, and the other one thereof is a preparation method that uses a 4-halo-phenylalanine derivative. However, these methods are disadvantageous because the palladium catalyst, which is expensive and difficult to handle, has to be used.

Also, a method of preparing acyl 4,4'-biphenylalanine having optical activity is disclosed, in which acyl 4,4'-biphenylalanine is subjected to optical resolution using chiral amine as an optical resolving agent to form a salt, which is then hydrolyzed (European Patent No. 2,387,558 (2010)), but the accurate optical purity of the obtained acyl 4,4'-biphenylalanine is not described.

### Disclosure

### Technical Problem

The present invention has been made keeping in mind the problems encountered in the related art.

Accordingly, an object of the present invention is to provide a method of preparing an optical isomer such as D-4,4'-biphenylalanine alkyl ester or L-4,4'-biphenylalanine alkyl ester having high optical purity by subjecting DL-4,4'-biphenylalanine alkyl ester to optical resolution using chiral diaroyl tartaric acid as an optical resolving agent.

### Technical Solution

In order to accomplish the above object,
the present invention provides a method of preparing D-4,4'-biphenylalanine alkyl ester of Chemical Formula 3 below or L-4,4'-biphenylalanine alkyl ester of Chemical Formula 4 below, comprising subjecting DL-4,4'-biphenylalanine alkyl ester of Chemical Formula 1 below to optical resolution using, as an optical resolving agent, chiral diaroyl tartaric acid of Chemical Formula 2 below. 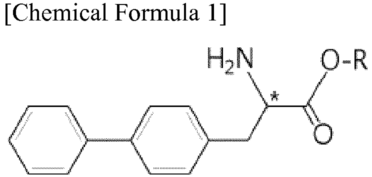 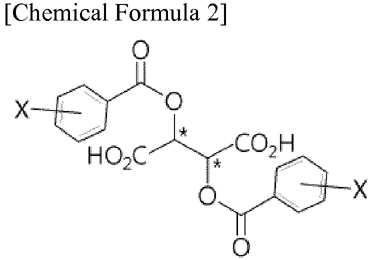 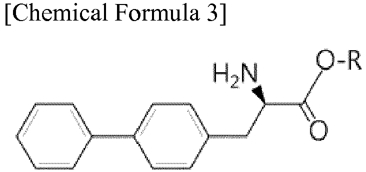 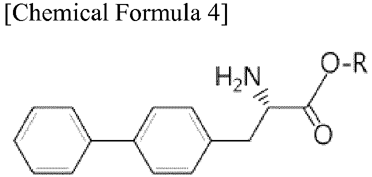
In Chemical Formulas 1, 3 and 4, R may be the same as or different from each other, and is a C1 to C4 alkyl group, and
* is chiral carbon.

In Chemical Formula 2, X is hydrogen, a C1 to C3 alkyl group, fluorine, chlorine, bromine, iodine, a cyano group or a nitro group.

In addition, the present invention provides a salt of D-4,4'-biphenylalanine alkyl ester and chiral diaroyl-D-tartaric acid, represented by Chemical Formula 7 below. 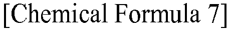 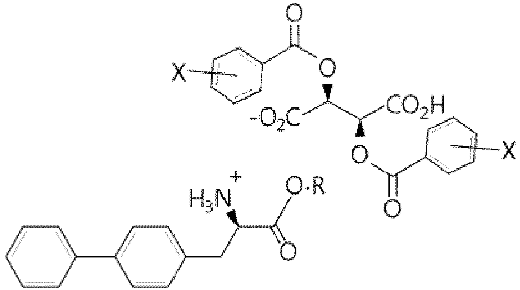
In Chemical Formula 7, R is a C1 to C4 alkyl group, and
X is hydrogen, a C1 to C3 alkyl group, fluorine, chlorine, bromine, iodine, a cyano group or a nitro group.

In addition, the present invention provides a salt of L-4,4'-biphenylalanine alkyl ester and chiral diaroyl-L-tartaric acid, represented by Chemical Formula 8 below. 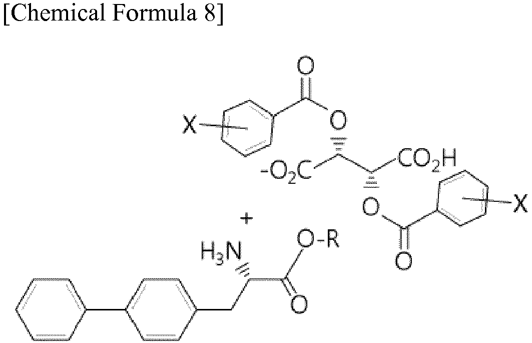
In Chemical Formula 8, R is a C1 to C4 alkyl group, and
X is hydrogen, a C1 to C3 alkyl group, fluorine, chlorine, bromine, iodine, a cyano group or a nitro group.

### Advantageous Effects

According to the present invention, a method of preparing D-4,4'-biphenylalanine alkyl ester or L-4,4'-biphenylalanine alkyl ester enables the preparation of D-4,4'-biphenylalanine alkyl ester or L-4,4'-biphenylalanine alkyl ester having high optical purity.

Also, according to the present invention, chiral diaroyl tartaric acid, which is an optical resolving agent used for the method of preparing D-4,4'-biphenylalanine alkyl ester or L-4,4'-biphenylalanine alkyl ester, is inexpensive, thus generating economic benefits.

Also, according to the present invention, the method of preparing D-4,4'-biphenylalanine alkyl ester or L-4,4'-biphenylalanine alkyl ester is very easy and is thus readily applicable and enables mass production.

### Brief Description of Drawings

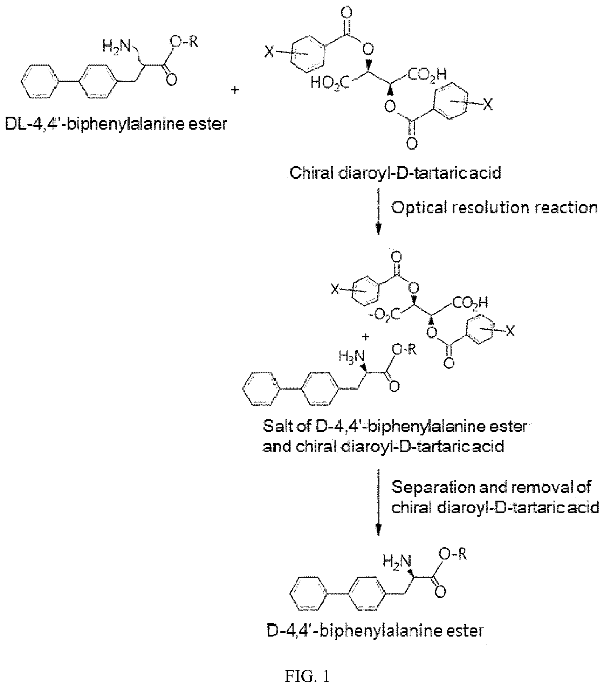

FIG. 1 schematically shows a process of preparing D-4,4'-biphenylalanine alkyl ester; and

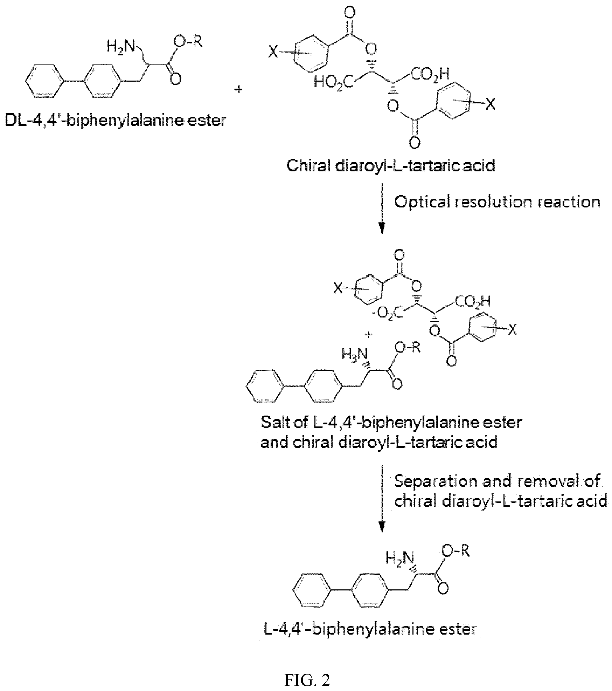

FIG. 2 schematically shows a process of preparing L-4,4'-biphenylalanine alkyl ester.

### Best Mode

Hereinafter, a detailed description will be given of the present invention.

Generally, a method of preparing an optically active compound using a chiral optical resolving agent is advantageous in that chemical processing is easily performed using a more simple apparatus compared to that used for biological processing, and is thus suitable for mass production.

However, searching for and selecting chiral compounds that are suitable for use as the optical resolving agent is regarded as very important, but is difficult.

According to the present invention, in order to separate an optical isomer mixture, namely DL-4,4'-biphenylalanine alkyl ester, into D-4,4'-biphenylalanine alkyl ester or L-4,4'-biphenylalanine alkyl ester having high optical purity, chiral diaroyl tartaric acid is adopted as an optical resolving agent, thus culminating in the present invention.

When chiral diaroyl tartaric acid is used as the optical resolving agent for optically resolving DL-4,4'-biphenylalanine alkyl ester, D-4,4'-biphenylalanine alkyl ester or L-4,4'-biphenylalanine alkyl ester having high optical purity may be obtained.

Specifically, the present invention pertains to a method of preparing D-4,4'-biphenylalanine alkyl ester of Chemical Formula 3 below or L-4,4'-biphenylalanine alkyl ester of Chemical Formula 4 below, comprising subjecting DL-4,4'-biphenylalanine alkyl ester of Chemical Formula 1 below to optical resolution using, as an optical resolving agent, chiral diaroyl tartaric acid of Chemical Formula 2 below. 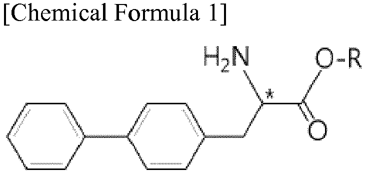 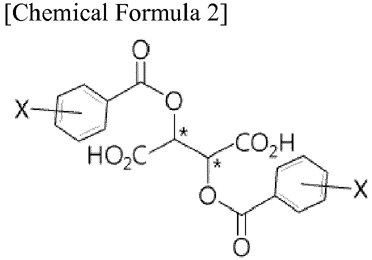 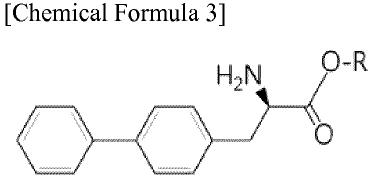 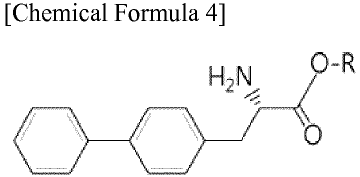
In Chemical Formulas 1, 3 and 4, R may be the same as or different from each other, and are a C1 to C4 alkyl group, and
* is chiral carbon.

In Chemical Formula 2, X is hydrogen, a C1 to C3 alkyl group, fluorine, chlorine, bromine, iodine, a cyano group or a nitro group.

As the optical resolving agent, chiral diaroyl tartaric acid of Chemical Formula 2 may be chiral diaroyl-D-tartaric acid of Chemical Formula 5 below or chiral diaroyl-L-tartaric acid of Chemical Formula 6 below.

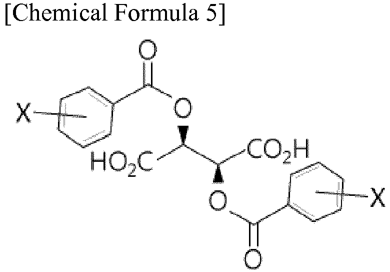

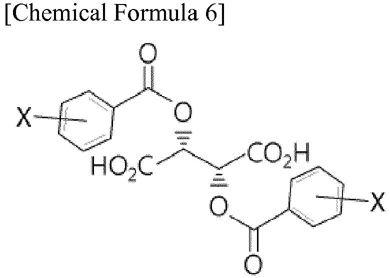

Here, X may be the same as or different from each other, and are hydrogen, a C 1 to C3 alkyl group, fluorine, chlorine, bromine, iodine, a cyano group or a nitro group.

More specifically, in the present invention, D-4,4'-biphenylalanine alkyl ester of Chemical Formula 3 may be prepared using chiral diaroyl-D-tartaric acid of Chemical Formula 5 as an optical resolving agent, and L-4,4'-biphenylalanine alkyl ester of Chemical Formula 4 may be prepared using chiral diaroyl-L-tartaric acid of Chemical Formula 6 as an optical resolving agent.

The optical resolution reaction is carried out in the presence of an organic solvent, and the organic solvent includes at least one selected from the group consisting of methanol, ethanol, isopropanol, ethyl acetate and toluene.

Upon the optical resolution reaction, DL-4,4'-biphenylalanine alkyl ester of Chemical Formula 1 and chiral diaroyl tartaric acid of Chemical Formula 2 are mixed at an equivalent ratio of 1:0.3 to 1:1.5, and preferably an equivalent ratio of 1:0.4 to 1:1.2.

If the equivalent ratio of chiral diaroyl tartaric acid of Chemical Formula 2 is less than 0.3, optical resolution efficiency may decrease. On the other hand, if the equivalent ratio thereof exceeds 1.5, optical resolution is not further progressed, thus negating economic benefits.

After the optical resolution, DL-4,4'-biphenylalanine alkyl ester of Chemical Formula 1 is extracted in the form of a salt, and the salt may be a salt of D-4,4'-biphenylalanine alkyl ester and chiral diaroyl-D-tartaric acid or a salt of L-4,4'-biphenylalanine alkyl ester and chiral diaroyl-L-tartaric acid, the salt being a solid.

The salt is present in the organic solvent, and may be separated using any process well-known in the art, for example, filtration, centrifugation or decantation. A filtration process is preferably used.

After separation of the salt from the organic solvent, chiral diaroyl-D-tartaric acid or chiral diaroyl-L-tartaric acid is removed from the salt, ultimately obtaining D-4,4'-biphenylalanine alkyl ester of Chemical Formula 3 or L-4,4'-biphenylalanine alkyl ester of Chemical Formula 4.

The separation and removal of the salt may be performed through any typically known process, particularly an extraction process using an organic solvent.

The solid salt obtained through optical resolution is added to a base aqueous solution, followed by extraction by the addition of at least one organic solvent selected from the group consisting of ethyl acetate and dichloromethane, whereby chiral diaroyl-D-tartaric acid or chiral diaroyl-L-tartaric acid is dissolved in the base aqueous solution and is thus removed, and D-4,4'-biphenylalanine alkyl ester of Chemical Formula 3 or L-4,4'-biphenylalanine alkyl ester of Chemical Formula 4 may be separated from the salt and may thus be finally obtained.

The kind of base aqueous solution is not particularly limited, but is an aqueous solution including at least one selected from the group consisting of sodium hydroxide, sodium carbonate and ammonia. Here, the pH thereof is preferably 9 to 12.

D-4,4'-biphenylalanine alkyl ester of Chemical Formula 3 or L-4,4'-biphenylalanine alkyl ester of Chemical Formula 4 thus prepared has optical activity, and the optical purity thereof may be analyzed using a chiral column.

D-4,4'-biphenylalanine alkyl ester of Chemical Formula 3 or L-4,4'-biphenylalanine alkyl ester of Chemical Formula 4 thus prepared has high optical purity, particularly optical purity of 85 to 99%.

The yield of D-4,4'-biphenylalanine alkyl ester of Chemical Formula 3 or L-4,4'-biphenylalanine alkyl ester of Chemical Formula 4 thus prepared is less than 50%. In order to increase the yield, a racemization catalyst may be further used during the optical resolution reaction in the present invention.

When the racemization catalyst is used in this way, optical resolution and racemization may occur simultaneously, thereby obtaining a yield of 50% or more.

The kind of racemization catalyst is not particularly limited, but preferably includes at least one selected from the group consisting of salicylaldehyde, 3,5-dichlorosalicylaldehyde, and 5-nitrosalicylaldehyde, and the use of 3,5-dichlorosalicylaldehyde is very preferable.

Also, R in Chemical Formulas 1, 3 and 4 is preferably a methyl group, and thus Chemical Formula 1 is preferably DL-4,4'-biphenylalanine methyl ester, Chemical Formula 3 is preferably D-4,4'-biphenylalanine methyl ester, and Chemical Formula 4 is preferably L-4,4'-biphenylalanine methyl ester.

Also, X in Chemical Formula 2 is preferably hydrogen or a p-methyl group, and Chemical Formula 2 may include at least one selected from the group consisting of di-p-toluoyl-D-tartaric acid, dibenzoyl-D-tartaric acid, di-p-toluoyl-L-tartaric acid, and dibenzoyl-L-tartaric acid.

Also, the present invention pertains to a salt of D-4,4'-biphenylalanine alkyl ester and chiral diaroyl-D-tartaric acid, represented by Chemical Formula 7 below. 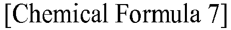 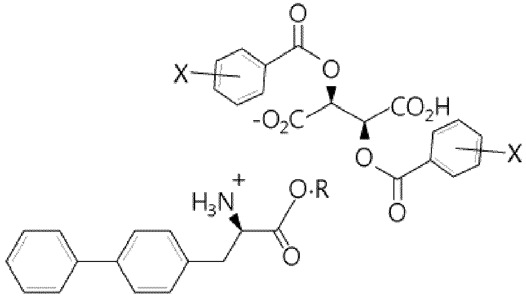
Here, R is a C1 to C4 alkyl group, and
X is hydrogen, a C1 to C3 alkyl group, fluorine, chlorine, bromine, iodine, a cyano group or a nitro group.

Preferably, R is a methyl group, and X is hydrogen or a p-methyl group.

Thus, the salt of Chemical Formula 7 may be a salt of D-4,4'-biphenylalanine methyl ester and di-p-toluoyl-D-tartaric acid or a salt of D-4,4'-biphenylalanine methyl ester and dibenzoyl-D-tartaric acid.

In addition, the present invention pertains to a salt of L-4,4'-biphenylalanine alkyl ester and chiral diaroyl-L-tartaric acid, represented by Chemical Formula 8 below. 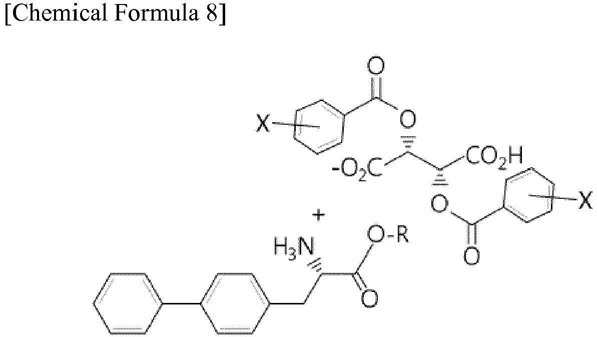
Here, R is a C1 to C4 alkyl group, and
X is hydrogen, a C1 to C3 alkyl group, fluorine, chlorine, bromine, iodine, a cyano group or a nitro group.

Preferably, R is a methyl group, and X is hydrogen or a p-methyl group.

Thus, the salt of Chemical Formula 8 may be a salt of L-4,4'-biphenylalanine methyl ester and di-p-toluoyl-L-tartaric acid or a salt of L-4,4'-biphenylalanine methyl ester and dibenzoyl-L-tartaric acid.

### Mode for Invention

A better understanding of the present invention will be given through the following examples, which are merely set forth to illustrate the present invention, but are not to be construed as limiting the scope of the present invention.

### Preparation Example 1. Preparation of DL-4,4'-biphenylalanine methyl ester

500 mL of methanol was added with 50 g of DL-4,4'-biphenylalanine and then with 49.3 g of thionyl chloride at 0°C, heated to 75°C with stirring for 3 hr, and then slowly cooled to room temperature. The solution was concentrated, thus obtaining 60.3 g of DL-4,4'-biphenylalanine methyl ester hydrochloride.

The DL-4,4'-biphenylalanine methyl ester hydrochloride was analyzed using ¹H-NMR. The results are as follows.

¹H-NMR (DMSO-d₆, 400 MHz): δ 3.18 (m, 2H), 3.68 (s, 3H), 4.29 (t, 1H), 7.33 (m, 3H), 7.45 (t, 2H), 7.63 (t, 4H), 8.69 (br, 3H).

The DL-4,4'-biphenylalanine methyl ester hydrochloride was added with 250 mL of water, 350 mL of dichloromethane, and 14 g of potassium hydroxide, and stirred for 1 hr. Thereafter, the dichloromethane layer was concentrated, thus obtaining 59.1 g of DL-4,4'-biphenylalanine methyl ester.

The DL-4,4'-biphenylalanine methyl ester thus obtained was analyzed using ¹H-NMR. The results are as follows.

¹H-NMR (D₂O, 400 MHz): δ 1.81 (br, 2H), 2.81 (m, 2H), 3.58 (t, 4H), 7.26 (d, 2H), 7.32 (t, 1H), 7.43 (t, 2H), 7.56 (d, 2H), 7.63 (d, 2H).

The optical purity of the salts obtained in the following examples was analyzed using a chiral column, and the analysis conditions are as follows.

Column: Chiralpak AD-H
Mobile phase: 10% IPA, 90% n-Hexane, 0.01% Diethylamine
Detector: UV (254 nm)

### Example 1. Preparation of D-4,4'- biphenylalanine methyl ester

150 mL of methanol was added with 10 g of DL-4,4'-biphenylalanine methyl ester of Preparation Example 1 and 7.6 g of di-p-toluoyl-D-tartaric acid, heated to 70°C with stirring for 2 hr, and then slowly cooled to room temperature.

Thereafter, filtration was performed, thus obtaining 8.3 g of a precipitated salt of D-4,4'-biphenylalanine methyl ester and di-p-toluoyl-D-tartaric acid (L/D ratio = 10/90).

The salt of D-4,4'-biphenylalanine methyl ester and di-p-toluoyl-D-tartaric acid was analyzed using ¹H-NMR. The results are as follows.

¹H-NMR (DMSO-d₆, 400 MHz): δ 2.34 (s, 6H), 3.04 (d, 2H), 3.23 (s, 3H), 4.13 (t, 1H), 5.63 (s, 2H), 7.27 (m, 6H), 7.34 (t, 1H), 7.45 (t, 2H), 7.58 (m, 4H), 7.81(d, 4).

The salt of D-4,4'-biphenylalanine methyl ester and di-p-toluoyl-D-tartaric acid was added with 41.5 mL of water, 58.1 mL of dichloromethane, and 2.5 g of potassium hydroxide, and stirred for 30 min. Thereafter, the dichloromethane layer was concentrated, thus yielding 3.2 g of D-4,4'-biphenylalanine methyl ester (L/D ratio = 10/90).

### Example 2. Preparation of D-4,4'-biphenylalanine methyl ester

150 mL of methanol and 75 mL of ethyl acetate were added with 15.0 g of DL-4,4'-biphenylalanine methyl ester of Preparation Example 1, 23.2 g of dibenzoyl-D-tartaric acid, and 0.56 g of 3,5-dichlorosalicylaldehyde, heated to 65°C with stirring for 20 hr, and then slowly cooled to room temperature.

Thereafter, filtration was performed, thus obtaining 23.4 g of a precipitated salt of D-4,4'-biphenylalanine methyl ester and dibenzoyl-D-tartaric acid (L/D ratio = 8/92).

The salt of D-4,4'-biphenylalanine methyl ester and dibenzoyl-D-tartaric acid thus obtained was analyzed using ¹H-NMR. The results are as follows.

¹H-NMR (DMSO-d₆, 400 MHz): δ 3.09 (m, 2H), 3.64 (s, 3H), 4.14 (t, 1H), 5.67 (s, 2H), 7.28 (d, 2H), 7.34 (t, 1H), 7.48 (m, 6H), 7.62 (m, 6H), 7.92 (d, 4H).

The salt of D-4,4'-biphenylalanine methyl ester and dibenzoyl-D-tartaric acid was added with 117 mL of water, 163.8 mL of dichloromethane, and 7.5 g of potassium hydroxide and stirred for 1 hr. Thereafter, the dichloromethane layer was concentrated, thus yielding 9.6 g of D-4,4'-biphenylalanine methyl ester (L/D ratio = 8/92).

### Example 3. Preparation of L-4,4'-biphenylalanine methyl ester

200 mL of methanol and 100 mL of isopropyl alcohol were added with 20.0 g of DL-4,4'-biphenylalanine methyl ester of Preparation Example 1, 19.6 g of dibenzoyl-L-tartaric acid, and 0.75 g of 3,5-dichlorosalicylaldehyde, and heated to 65°C with stirring for 20 hr, and then slowly cooled to room temperature.

Thereafter, filtration was performed, thus obtaining 26.4 g of a precipitated salt of L-4,4'-biphenylalanine methyl ester and dibenzoyl-L-tartaric acid (L/D ratio = 95/5).

The salt of L-4,4'-biphenylalanine methyl ester and dibenzoyl-L-tartaric acid was analyzed using ¹H-NMR. The results are as follows.

¹H-NMR (DMSO-d₆, 400 MHz): δ 3.11 (m, 2H), 3.64 (s, 3H), 4.13 (t, 1H), 5.67 (s, 2H), 7.28 (d, 2H), 7.34 (t, 1H), 7.48 (m, 6H), 7.62 (m, 6H), 7.92 (d, 4H).

The salt of L-4,4'-biphenylalanine methyl ester and dibenzoyl-L-tartaric acid was added with 132 mL of water, 184 mL of dichloromethane, and 8.4 g of potassium hydroxide and stirred for 1 hr.

Thereafter, the dichloromethane layer was concentrated, thus yielding 10.6 g of L-4,4'-biphenylalanine methyl ester (10.6 g, L/D ratio = 95/5).

## Claims

1. A method of preparing D-4,4'-biphenylalanine alkyl ester of Chemical Formula 3 below or L-4,4'-biphenylalanine alkyl ester of Chemical Formula 4 below, comprising subjecting DL-4,4'-biphenylalanine alkyl ester of Chemical Formula 1 below to optical resolution using, as an optical resolving agent, chiral diaroyl tartaric acid of Chemical Formula 2 below: 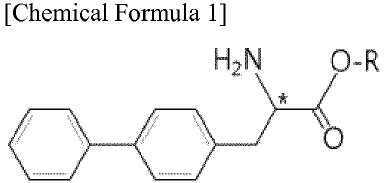 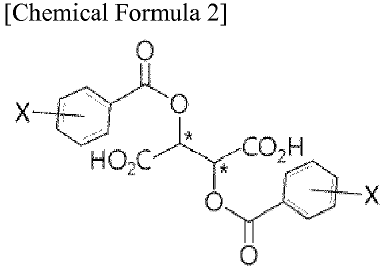 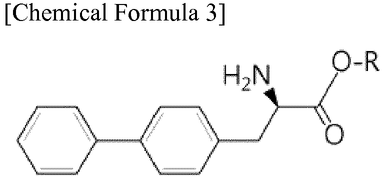 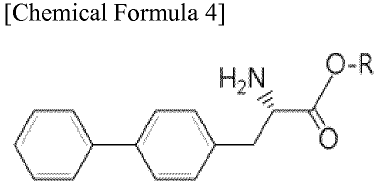 in Chemical Formulas 1, 3 and 4,
R is the same as or different from each other and are a C1 to C4 alkyl group, and
* is chiral carbon, and
in Chemical Formula 2,
X is hydrogen, a C1 to C3 alkyl group, fluorine, chlorine, bromine, iodine, a cyano group or a nitro group.

2. The method of claim 1, wherein the chiral diaroyl tartaric acid of Chemical Formula 2 as the optical resolving agent is chiral diaroyl-D-tartaric acid of Chemical Formula 5 below or chiral diaroyl-L-tartaric acid of Chemical Formula 6 below: 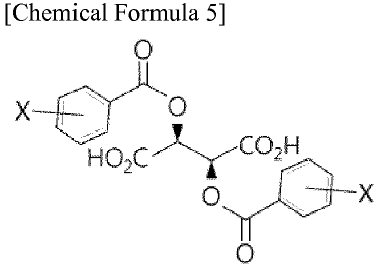 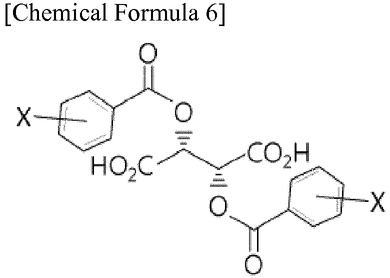 wherein X is the same as or different from each other and are hydrogen, a C1 to C3 alkyl group, fluorine, chlorine, bromine, iodine, a cyano group or a nitro group.

3. The method of claim 2, wherein the D-4,4'-biphenylalanine alkyl ester of Chemical Formula 3 is prepared using the chiral diaroyl-D-tartaric acid of Chemical Formula 5 as the optical resolving agent.

4. The method of claim 2, wherein the L-4,4'-biphenylalanine alkyl ester of Chemical Formula 4 is prepared using the chiral diaroyl-L-tartaric acid of Chemical Formula 6 as the optical resolving agent.

5. The method of claim 1, wherein the optical resolution is carried out using a solvent including at least one selected from the group consisting of methanol, ethanol, isopropanol, ethyl acetate, and toluene.

6. The method of claim 1, wherein the DL-4,4'-biphenylalanine alkyl ester of Chemical Formula 1 and the chiral diaroyl tartaric acid of Chemical Formula 2 are mixed at an equivalent ratio of 1:0.3 to 1:1.5.

7. The method of claim 2, wherein the DL-4,4'-biphenylalanine alkyl ester of Chemical Formula 1 is precipitated in a salt form after the optical resolution.

8. The method of claim 7, wherein the salt is a salt of D-4,4'-biphenylalanine alkyl ester and chiral diaroyl-D-tartaric acid or a salt of L-4,4'-biphenylalanine alkyl ester and chiral diaroyl-L-tartaric acid.

9. The method of claim 7, wherein D-4,4'-biphenylalanine alkyl ester or L-4,4'-biphenylalanine alkyl ester is obtained by removing chiral diaroyl-D-tartaric acid or chiral diaroyl-L-tartaric acid from the salt using an organic solvent including at least one selected from the group consisting of ethyl acetate and dichloromethane.

10. The method of claim 1, wherein a racemization catalyst is further used during the optical resolution.

11. The method of claim 10, wherein the racemization catalyst includes at least one selected from the group consisting of salicylaldehyde, 3,5-dichlorosalicylaldehyde, and 5-nitrosalicylaldehyde.

12. The method of claim 1, wherein R of Chemical Formulas 1, 3 and 4 is a methyl group.

13. The method of claim 1, wherein X of Chemical Formula 2 is hydrogen or a p-methyl group.

14. The method of claim 13, wherein the Chemical Formula 2 includes at least one selected from the group consisting of di-p-toluoyl-D-tartaric acid, dibenzoyl-D-tartaric acid, di-p-toluoyl-L-tartaric acid, and dibenzoyl-L-tartaric acid.

15. A salt of D-4,4'-biphenylalanine alkyl ester and chiral diaroyl-D-tartaric acid, represented by Chemical Formula 7 below: 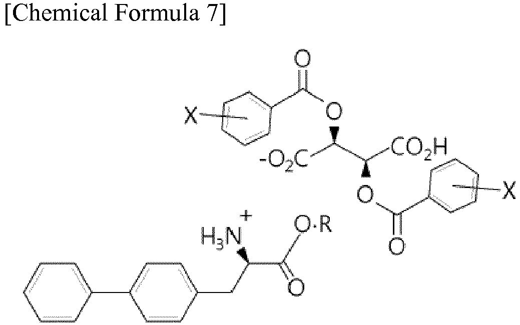
wherein R is a C1 to C4 alkyl group, and
X is hydrogen, a C1 to C3 alkyl group, fluorine, chlorine, bromine, iodine, a cyano group or a nitro group.

16. The salt of claim 15, wherein the salt is a salt of D-4,4'-biphenylalanine methyl ester and di-p-toluoyl-D-tartaric acid or a salt of D-4,4'-biphenylalanine methyl ester and dibenzoyl-D-tartaric acid.

17. A salt of L-4,4'-biphenylalanine alkyl ester and chiral diaroyl-L-tartaric acid, represented by Chemical Formula 8 below: 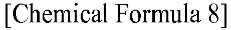 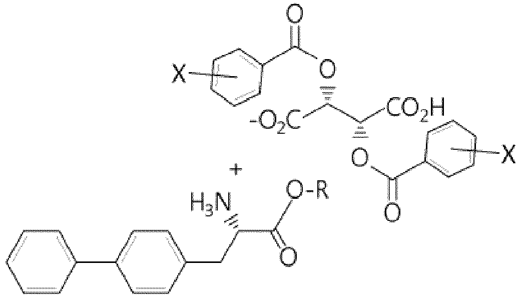
wherein R is a C1 to C4 alkyl group, and
X is hydrogen, a C1 to C3 alkyl group, fluorine, chlorine, bromine, iodine, a cyano group or a nitro group.

18. The salt of claim 17, wherein the salt is a salt of L-4,4'-biphenylalanine methyl ester and di-p-toluoyl-L-tartaric acid or a salt of L-4,4'-biphenylalanine methyl ester and dibenzoyl-L-tartaric acid.
